(19) Europäisches Patentamt
European Patent Office
Office européen des brevets

(11) Publication number: 0 171 092
B1

(12) EUROPEAN PATENT SPECIFICATION

(45) Date of publication of patent specification: 30.08.89

(51) Int. Cl.$^4$: C 07 C 149/12, C 07 C 148/00

(21) Application number: 85113184.7

(22) Date of filing: 17.10.85

# (54) Preparation of dialkyl disulfides.

(30) Priority: 26.04.85 US 727706

(43) Date of publication of application:
12.02.86 Bulletin 86/07

(45) Publication of the grant of the patent:
30.08.89 Bulletin 89/35

(84) Designated Contracting States:
AT BE CH DE FR GB IT LI LU NL SE

(58) References cited:
US-A-4 302 605

(73) Proprietor: PENNWALT CORPORATION
Pennwalt Building Three Parkway
Philadelphia Pennsylvania 19102 (US)

(72) Inventor: Custer, Robert Sloan
165 Biddulph Road
Radnor Pennsylvania 19087 (US)
Inventor: Haines, Paul Gordon
3038 Joshua Road
Lafayette Hill Pennsylvania (US)

(74) Representative: Kraus, Walter, Dr. et al
Patentanwälte Kraus, Weisert & Partner
Thomas-Wimmer-Ring 15
D-8000 München 22 (DE)

The file contains technical information submitted after the application was filed and not included in this specification

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

EP 0 171 092 B1

Courier Press, Leamington Spa, England.

**Description**

This invention relates to a method for preparing dialkyl disulfides by reacting an alkyl alcohol, hydrogen sulfide and sulfur in the presence of a catalyst. More particularly, it relates to the preparation of dialkyl disulfides in a single reaction zone in accordance with the equation:

(1) $$2ROH + S + H_2S \rightarrow RSSR + 2H_2O$$

Dialkyl disulfides are usually produced by oxidation of an alkyl mercaptan with air or oxygen according to the following equation:

(2) $$2RSH + \tfrac{1}{2}O_2 \rightarrow RSSR + H_2O$$

Vapor-phase reactions of the type shown in equation (2) are disclosed by E. E. Reid in "Organic Chemsitry of Bivalent Sulfur" Volume 1, p. 118 (1958) and in U.S. Patent No. 2,979,532.

It is also known to prepare dialkyl dusulfides by reacting in the liquid phase an alkyl mercaptan with sulfur in accordance with equation (3).

(3) $$2RSH + S \rightarrow RSSR + H_2S$$

This type of reaction is disclosed in U.S. Patents No. 3,299,146 and 3,314,999.

The preparation of alkyl mercaptans from alkyl alcohols and hydrogen sulfide according to equation (4) is also known.

(4) $$ROH + H_2S \rightarrow RSH + H_2O$$

This reaction is disclosed in U.S. 3,035,097.

Until now, it has been necessary to first prepare and isolate the mercaptan in one reaction [equation (4)] with a specified catalyst and then to oxidize the isolated mercaptan with oxygen [equation (2)] or sulfur [equation (3)] in a separate process under different conditions and with a different catalyst.

Finally, it is known in the art to prepare dialkyl sulfides by reacting an alkyl alcohol with hydrogen sulfide in accordance with equation (5).

(5) $$2ROH + H_2S \rightarrow RSR + 2H_2O$$

The process in which the reaction of equation (5) is carried out is shown in U.S. patent No. 4 302 605 and includes the use as a catalyst of a Type X, Y or L zeolite. In the process shown in the patent, a minor amount of dialkyl disulfide is obtained as a heavy bottoms stream.

This invention relates to a process for the continuous preparation of a di($C_1$—$C_{12}$) alkyl disulfide comprising continuously feeding into a reaction zone a $C_1$—$C_{12}$ alkyl alcohol and hydrogen sulfide at a temperature ranging from 200°C to 400°C, at a pressure ranging from atmospheric to 42.3 bar (600 psig) and in the presence of a solid, particulate catalyst whereby a di($C_1$—$C_{12}$) alkyl disulfide is continuously produced, which is characterized by continuously feeding into the reaction zone elemental sulfur at a mol ratio of $C_1$—$C_{12}$ alkyl alcohol, hydrogen sulfide and elemental sulfur ranging, respectively, from 2/1/0.5 to 1/10/10. The process is improved by recovering any alkyl mercaptan formed during the reaction and recycling it to the reaction zone.

A preferred process according to the invention is a process for the continuous preparation of dimethyl disulfide comprising continuously feeding into a reaction zone methanol, hydrogen sulfide, recycled mercaptan and an inert gas at a temperature ranging from 250 to 350°C, at a pressure of from 7.9 to 32 bar (100 to 450 psig) and at a molar velocity of 50 to 150, and in the presence of a catalyst selected from the group consisting of Type Y zeolites, alumina bearing phosphotungstic acid or its alkali metal salt, and alumina bearing silicotungstic acid or its alkali metal salt, which is characterized by continuously feeding into the reaction zone elemental sulfur at a mol ratio of methanol, hydrogen sulfide, elemental sulfur, recycled mercaptan and an inert gas ranging, respectively, from 1/1/0.5/2/1 to 1/10/6/10/15.

The process of this invention provides for the more economical and continuous manufacture of dialkyl disulfides in a single reaction zone.

In the process, an alkyl alcohol, hydrogen sulfide and sulfur are mixed continuously in appropriate molar ratios and the mixture contacted with a solid particulate catalyst at a sufficiently high temperature to continuously produce a dialkyl disulfide product in good yields.

The process can be illustrated by the equation:

(1) $$2ROH + S + H_2S \rightarrow RSSR + 2H_2O$$

ROH in the above equation represents a straight chain, branched chain or cyclic $C_1$—$C_{12}$ alkyl alcohol including, for example, methyl, ethyl, propyl, isopropyl, butyl, isobutyl, tertiary butyl, amyl, isoamyl, hexyl,

cyclohexyl, octyl, isooctyl, decyl, dodecyl and isomeric forms of these alcohols. Preferably, the alcohol is a straight-chain or branched chain $C_1$ to $C_4$ alkanol. Most preferably, the alkanol is methanol.

Sulfur (S) and hydrogen sulfide ($H_2S$) for this process can be obtained from any source providing a reasonably pure product.

The reaction is effected at a sufficiently high temperature to produce a reasonable yield of the dialkyl disulfide product. The process temperature is gauged by the temperature of the catalyst bed which generally ranges between 200 and 400°C, and is preferably kept within the range of 250 and 350°C.

The pressure at which the process is operated is generally from atmospheric to 42.3 bar (600 psig) or higher, preferably 7.9 to 32 bar (100 to 450 psig).

The reactants are continuously fed to the reactor either premixed or in metered amounts so that the molar ratio of reactants in the order ROH/$H_2$S/S respectively range from 2/1/0.5 to 1/10/10. In the preferred embodiment of the invention any alkyl mercaptan (RSH) formed is recycled to the reactor so that the molar ratio of reactants (including recycled RSH) in the order ROH/$H_2$S/S/RSH respectively range from 2/1/1/1 to 1/10/10/10. The preferred molar ratio of ROH/$H_2$S/S/RSH entering the reactor ranges respectively from 1/1/0.5/2 to 1/10/6/10.

In addition to the reactants, the feed stream entering the reactor may contain an inert gas such as methane, carbon dioxide, or nitrogen as necessary to remove heat from the reaction zone. The molar ratio of inert gas to alkanol entering the reactor may range from 0/1 to about 15/1.

The rate at which dialkyl disulfide is produced is controlled by the velocity of reactants over the catalyst, which is measured by the amount of alkyl alcohol passing through the catalyst bed. The molar velocity for this process is defined as the number of pound-moles of alkanol reactant passing through 1000-pounds of catalyst per 24-hour day or the number of gram-moles of alkanol per kilogram of catalyst per 24 hour day. The molar velocity generally ranges from 20 to 200 and preferably from 50 to 150.

Any solid, particulate catalyst which will affect the reaction of an alkanol with sulfur and hydrogen sulfide to produce a dialkyl disulfide in good yields is useful for this process. Several catalysts have been found particularly useful. One useful catalyst group is the zeolite (molecular sieve) catalysts which are synthetic aluminosilicates characterized by high uniformity, well-defined pore size, large surface area, complete crystallinity, and excellent reproducibility. Their structures are described in the Union Carbide booklet F-08 entitled, "Linde Molecular Sieve Catalysts" and D. W. Breck's, "Zeolite Molecualr Sieves", John Wiley & Sons (1974). Various types are currently marketed by Union Carbide, Houdry (Air Products and Chemicals), Davison (W. R. Grace), Norton, and Akzo Chemie.

The basic structural units, framework structure and pore size of synthetic zeolites are more specifically described in the patent art, for example, U.S. Patent No. 4,302,605 (Column 2, lines 15—65), incorporated herein by reference. Additionally, Type L is further defined as more siliceous than Type X and Type Y, and also having a pore size in the 7 to 10 Angstrom range.

The preferred zeolites are the Type Y, due to their greater thermal stability. The most preferred zeolites are Type Y in which the sodium cation has been exchanged with ammonium cation, and the zeolite has then been calcined at temperatures above 500°C to remove ammonia, leaving essentially a protonated zeolite catalyst. Zeolites of this type are marketed by Union Carbide Corporation under the designations LZ-Y62, LZ-Y72, and LZ-Y82.

Another catalyst group which is useful for the process of this invention is the heteropoly acids and their alkali or alkaline earth metal salts deposited on a particulate metal oxide support.

The heteropoly acids and their salts are well-known compounds. They are defined by Sidgewick in his book, "The Chemical Elements and Their Compounds", Vol. 11, page 1042 (1940), as those complicated structures in which a large number (usually 6, 9 or 12) of molybdic or tungstic acid residues are combined with a single residue of another acid selected from the group of oxy-acids of boron, silicon, germanium, titanium, zirconium, thorium, phosphorus, vanadium, arsenic and manganese. A more complete description of the heteropolyacids, their salts, metal oxide supports therefor, and methods of preparation are shown in patents and technical literature, for example, U.S. Patent No. 3,035,097 (Column 2 line 43 through Column 4 line 24), incorporated herein by reference.

It is found that best results are obtained with those heteropoly acids containing tungsten and the preferred catalysts for this process are phosphotungstic acid and silicotungstic acid or their alkali metal salts deposited on an inert metal oxide support. The preferred support (dehydration catalyst base) is alumina.

Other supports such as titania, silica, chromia or other oxides such as the oxides of tungsten, uranium, molybdenum, etc, are also useful supports. The amount of heteropoly acid (or its salt) deposited on the support will be such that from 0.1% to 10% (preferably from 1% to 8%) by weight of the final dry catalyst composite is the heteropoly acid compound. When the amount of heteropoly acid is above 10%, the process is operable, but the conversions are no higher. The most preferred catalyst of this type contains 2—8% by weight of potassium phosphotungstate on an alumina support.

The catalysts are used in this process in the form of beds over or through which the reactants are passed. When spent, the catalyst is either replaced or regenerated, if possible.

The end products of the process of this invention are useful for the pretreatment of hydrodesulfurization catalysts, as downhole sulfur solvents in oil well drilling and as substitutes for alkyl mercaptans in chemical processes.

THE DRAWING

The drawing is a flow diagram depicting the manufacture of di-$C_1$—$C_4$ alkyl disulfide. A $C_1$—$C_4$ alkyl alcohol, hydrogen sulfide and elemental sulfur are fed continuously in a molar ratio of about 2:1:1 through vaporizing heaters 10, 12 and 14, mixed and then passed into the reactor 16 containing the catalyst bed. Elevated temperatures and pressures, in accordance with this disclosure, are used to effect the reaction. The crude product stream from the reactor is cooled and passed into a sulfur knockout pot 18 to remove unreacted sulfur. The unreacted sulfur is passed via line 20 back to reactor 16 after reheating. The crude reaction product from pot 18 is cooled further and passed into a water-separator 22 where the by-product water is removed from the organics by a liquid-phase layer separation. The crude organic product is then pased through a series of distillation columns. The first column 24 removes the low boiling components including hydrogen sulfide, carbon dioxide, carbonyl sulfide, inert gases (coolants) and at least a portion of $C_1$—$C_4$ alkyl mercaptan by-product in an overhead stream which is recycled to the reactor 16 through line 26. The crude product bottoms stream from column 24 is fed through line 28 to a second distillation column 30 where the remainder of the low boilers, the $C_1$—$C_4$ alkyl mercaptan, some by product di($C_1$—$C_4$)alkyl sulfide and unreacted $C_1$—$C_4$ alkyl alcohol are removed overhead and recycled to the reactor through line 26.

The crude product bottoms stream from column 30, containing di($C_1$—$C_4$) alkyl disulfide, by product di($C_1$—$C_4$)alkyl sulfide, and high boiling by-products consisting mainly of di($C_1$—$C_4$)alkyl polysulfides having 3 to 5 sulfur atoms is passed to column 34 through line 32. Di($C_1$—$C_4$)alkyl sulfide is separated as an overhead product from distillation column 34 through line 36. Alternatively, the by product di($C_1$—$C_4$)alkyl sulfide can be reheated and recycled to reactor 16, where it will build up to a certain equilibrium level in the recycle, on which level there is no further net increase in sulfide-formation across the reactor. The bottoms stream from column 34, containing di($C_1$—$C_4$)alkyl disulfide and di($C_1$—$C_4$)alkyl polysulfides is passed through bottom line 38 to column 40. In column 40, high-purity di($C_1$—$C_4$)alkyl disulfide is obtained as an overhead product (line 42) and the di($C_1$—$C_4$)alkyl polysulfides are obtained as a bottoms product through line 44. Alternatively, the di($C_1$—$C_4$)alkyl polysulfides (line 44) can be reheated and recycled to reactor 16, where they will react with the $C_1$—$C_4$ mercaptan present in the reactor, in a disproportionation reaction, to produce additional desired di($C_1$—$C_4$)alkyl disulfide.

## Examples

An experimental reactor was used to provide the following examples demonstrating the process of this invention, as reported below. The reactor consisted of a 5.08 cm (2 inches) in diameter 101.6 cm (40 inches) long 316 stainless steel tube enclosed in an electrically heated horizontal furnace. 10.16 cm (4 inches) of the reactor tube extending completely within the confines of the horizontal furnace is packed with the solid particulate catalyst. Feed gases nitrogen (for temperature control) and hydrogen sulfide were fed to the reactor through metering valves into a preheater furnace, heated to 200°C and then into a mixing manifold situated at the front of the reactor. Alkanol and "recycle" alkyl mercaptan (simulated) were pumped from a reservoir mounted with flexible couplings on continuous weight scales. Molten sulfur is supplied from a heat-jacketed pump. Alkanol, hydrogen sulfide, molten sulfur and nitrogen gas are fed at a specified molar ratio and molar velocity to the mouth of the reactor tube for passage through the catalyst in the reactor. The crude reaction product exiting the reactor and an adjacent sulfur knockout pot was passed as a vapor into the gas-sampling device of a gas chromatograph by means of an electrically-traced 316 stainless steel tubing through a back-pressure control release valve regulated to maintain an operating pressure of 11.3 bar (150 psig). The conversion to dialkyl disulfide (DMDS) was calculated from the gas chromatographic analysis.

### Example 1

In two separate runs, A and B, the reactants, methanol, $H_2S$, sulfur and "recycle" methyl mercaptan along with inert nitrogen gas were fed into the above described reactor under the conditions specified above at a molar ratio respectively of 1/2/4/9/10 and at other conditions reported in the following table. The catalyst used in the reactor was an acid (protonated) Type Y zeolite (Union Carbide's LZ-Y62) prepared by exchanging the sodium cations on a zeolite originally containing about 13 percent by weight sodium, expressed as $Na_2O$, with ammonium cations and calcining the resulting zeolite at 500°C to remove ammonia and leave essentially a protonated zeolite containing only 2.5 percent by weight of sodium, expressed as $Na_2O$. The experimental results are shown in Table 1, in which the last column shows the conversion, in a single pass, of methanol and methyl mercaptan combined to dimethyl disulfide (DMDS), expressed on a percentage of the DMDS that is theoretically possible from these two feeds combined.

An examination of the data in Table 1 clearly shows that major portions of the methanol and the "recycle" methyl mercaptan that were fed to the reactor were consumed, and that the major product produced is DMDS. The consumption of the methanol feed and the "recycle" methyl mercaptan, with formation of substantial quantities of DMDS, can best be accounted for by postulating a simultaneous reaction of methanol and $H_2S$ to produce methyl mercaptan (equation 4) and of methyl mercaptan and sulfur to produce DMDS (equation 3), at the conditions existing in the reaction zone.

The major byproduct at these conditions is dimethyl sulfide. It was demonstrated that at least a portion of this byproduct can be recycled to the reaction zone without adverse effect on formation of DMDS.

**Table 1**

**LZ-Y62 ZEOLITE CATALYST**

| RUN | CONDITIONS | | INPUT(*) | | OUTPUT(*) | | | | | CONVERSION(**) |
|---|---|---|---|---|---|---|---|---|---|---|
| Run | Cat. Bed Temp. °C | $CH_3OH$ Molar Velocity | $CH_3OH$ | "Recycle" $CH_3SH$ | Recovered $CH_3OH$ | Recovered $CH_3SH$ | Byproduct $CH_3SCH_3$ | Byproduct $CS_2$ | DMDS | DMDS |
| A | 314 | 117 | 3748 | 47,147 | 645 | 16,176 | 5318 | 320 | 26,611 | 51.5 |
| B | 304 | 114 | 3652 | 46,185 | 1035 | 19,374 | 4616 | 923 | 22,518 | 44.5 |

* - Calculated as pounds/1000 lbs. catalyst/24 hour day (or grams/kilogram catalyst/24 hour day)

**- Percent of the theoretically-possible DMDS obtained from the methanol and "recycle" methyl mercaptan combined, in a single pass.

Example 2

In three (3) separate runs, C, D and E, the reactants of Example 1, at a molar ratio for $CH_3OH/H_2S/S/CH_3SH/N_2$ of 1/4/6/4/5, were fed to the above described reactor under the condition specified above and at other conditions described in the following Table 2. In addition, a separate reaction (Run F) was conducted with the same reactants, in the same reactor and under the same conditions described above except that the ratio of reactants respectively was 1/2/4/9/10. In all the runs of this Example, the catalyst employed was 5% by weight of potassium phosphotungstate (KPT) deposited on 95% by weight of alumina prepared by dissolving 6.3 g phosphotungstic acid in a mixture of 75 ml distilled water and 7.2 g of 45% aqueous potassium hydroxide solution. After stirring and warming almost to boiling, the solution was uniformly added portionwise to 170 g of F-1 alumina (Alcoa). The resulting catalyst was dried overnight at 150°C. The KPT content is approximately 5% of the catalyst weight.

The results in Table 2 show that almost all the methanol and a major portion of the "recycle" methyl mercaptan, were converted to products. The major product formed was DMDS, with some byproduct $CS_2$ and a minor amount of byproduct dimethyl sulfide also formed. These results can only be accounted for by a simultaneous conversion of methanol to methyl mercaptan (equation 4) and methyl mercaptan to DMDS (equation 3). This can be illustrated further by examining the data for Run D. Assuming, in Run D, for example, that all the byproduct dimethyl sulfide and all the byproduct $CS_2$ produced had come from the methanol and none from the more abundant methyl mercaptan, the 85.4 kg (188 lbs) of dimethyl sulfide and the 633.8 kg (1396 lbs) of $CS_2$ produced would have required 88 kg (194 lbs) and 267 kg (588 lbs) of methanol, respectively, totalling 355 kg (782 lbs) of methanol. The methanol input, however, was 756 kg (1666 lbs). Thus, the remaining 401 kg (884 lbs) of methanol, less the 32.7 kg (72 pounds) of unreacted methanol recovered, or at least 369 kg (812 lbs) of methanol, must have been converted to DMDS.

The major byproduct on these conditions is $CS_2$. It was demonstrated that the $CS_2$ can be totally recycled to the reaction zone, where it reacts readily with methanol to produce the desired intermediate product, methyl mercaptan.

Table 2

KPT - ALUMINA CATALYST

| RUN | CONDITIONS | | INPUT(*) | | OUTPUT(*) | | | | | CONVERSION(**) |
|---|---|---|---|---|---|---|---|---|---|---|
| | Cat. Bed Temp., °C | $CH_3OH$ Molar Velocity | $CH_3OH$ | "Recycle" $CH_3SH$ | Recovered $CH_3OH$ | Recovered $CH_3SH$ | By-Product $CH_3SCH_3$ | By-Product $CS_2$ | DMDS | DMDS |
| C | 309 | 50 | 1,602 | 9,622 | 25 | 3832 | 181 | 3748 | 5,328 | 45.3 |
| D | 314 | 51 | 1,666 | 9,622 | 72 | 5310 | 188 | 1396 | 5,183 | 43.7 |
| E | 291 | 48 | 1,634 | 9,622 | 150 | 4288 | 120 | 2657 | 5,987 | 50.7 |
| F | 311 | 100 | 3,204 | 48,110 | 1201 | 22,728 | 246 | 1141 | 26,637 | 51.4 |

* - Calculated as pounds/1000 lbs. catalyst/24 hour day (or grams/kilogram catalyst/24 hour day).

**- Percent of the theoretically-possible DMDS obtained from the methanol and "recycle" methyl mercaptan combined, in a single pass.

Example 3

Run G in Table 3 was carried out in the same manner as the runs in Example 2, except that Alcoa's F-1 alumina, without the deposited KPT, was used in place of the catalyst. The data clearly show that without the heteropoly acid salt as a promoter, the alumina is relatively ineffective for the desired reaction.

Table 3

F-1 ALUMINA CATALYST

| RUN | CONDITIONS | | INPUT(*) | | | OUTPUT(*) | | | | CONVERSION(**) |
|---|---|---|---|---|---|---|---|---|---|---|
| | Catalyst Bed Temp. °C | $CH_3OH$ Molar Velocity | $CH_3OH$ | "Recycle" $CH_3SH$ | Unconverted $CH_3OH$ | Recovered $CH_3SH$ | Byproduct $CH_3SCH_3$ | Byproduct $CS_2$ | DMDS | DMDS |
| G | 331 | 108 | 3460 | 24,055 | 1324 | 24,361 | 768 | 0 | 590 | 2.1 |

* - Calculated as pounds/1000 lbs. catalyst/24 hour day (or grams/kilogram catalyst/24 hour day).

** - $CH_3OH + CH_3SH \rightarrow (CH_3)_2S_2$ (percent of theoretical).

**Claims**

1. A process for the continuous preparation of a di($C_1$—$C_{12}$) alkyl disulfide comprising continuously feeding into a reaction zone a $C_1$—$C_{12}$ alkyl alcohol and hydrogen sulfide at a temperature ranging from 200°C to 400°C, at a pressure ranging from atmospheric to 42.3 bar (600 psig) and in the presence of a solid, particulate catalyst whereby a di($C_1$—$C_{12}$) alkyl disulfide is continuously produced, characterized by continuously feeding into the reaction zone elemental sulfur at a mol ratio of $C_1$—$C_{12}$ alkyl alcohol, hydrogen sulfide and elemental sulfur ranging, respectively, from 2/1/0.5 to 1/10/10.

2. The process of claim 1 wherein alkyl mercaptan produced during the reaction is recovered and recycled to said reaction zone.

3. The process of claim 2 wherein the alkyl alcohol is a $C_1$—$C_4$ alkyl alcohol and the molar velocity is from 20 to 200.

4. The process of claim 3 wherein the alcohol, hydrogen sulfide, elemental sulfur and recycled mercaptan are fed at a mol ratio ranging, respectively, from 1/1/0.5/2 to 1/10/6/10, the temperature ranges from 250 to 350°C, the pressure ranges from 7.9 to 32 bar (100 to 450 psig), and the molar velocity is 50 to 150

5. The process of claim 3 wherein the byproduct dialkyl sulfide is recycled to said reaction zone.

6. The process of claim 3 wherein the byproduct polysulfides are recycled to said reaction zone.

7. The process of claim 3 wherein the catalyst is a Type X, Y or L zeolite.

8. The process of claim 3 wherein the catalyst is a heteropoly acid or its alkali metal or alkaline earth metal salt deposited on an inert metal oxide support.

9. The process of claim 4 wherein the catalyst is a Type Y zeolite.

10. The process of claim 4 wherein the catalyst is a phosphotungstic alkali metal salt deposited on alumina.

11. A process for the continuous preparation of dimethyl disulfide comprising continuously feeding into a reaction zone methanol, hydrogen sulfide, recycled mercaptan and an inert gas at a temperature ranging from 250 to 350°C, at a pressure of from 7.9 to 32 bar (100 to 450 psig) and at a molar velocity of 50 to 150, and in the presence of a catalyst selected from the group consisting of Type Y zeolites, alumina bearing phosphotungstic acid or its alkali metal salt, and alumina bearing silico-tungstic acid or its alkali metal salt, characterized by continuously feeding into the reaction zone elemental sulfur at a mol ratio of methanol, hydrogen sulfide, elemental sulfur, recycled mercaptan and an inert gas ranging, respectively, from 1/1/0.5/2/1 to 1/10/6/10/15.

12. The process of claim 11 wherein the catalyst is a protonated Type Y zeolite.

13. The process of claim 11 wherein the catalyst is an alumina bearing alkali metal salt of phosphotungstic acid where the salt is from 1 to 8% based on the catalyst weight.

**Patentansprüche**

1. Verfahren zur kontinuierlichen Herstellung eines Di-($C_1$—$C_{12}$)-alkyldisulfids durch kontinuierliche Einspeisung eines $C_1$—$C_{12}$-Alkylalkohols und von Schwefelwasserstoff in eine Reaktionszone bei einer Temperatur im Bereich von 200 bis 400°C, bei einem Druck im Bereich von Atmosphärendruck bis 42,3 bar (600 psig) und in Gegenwart eines festen teilchenförmigen Katalysators, wodurch kontinuierlich ein Di-($C_1$—$C_{12}$)-alkyldisulfid hergestellt wird, dadurch gekennzeichnet, daß man kontinuierlich in die Reaktionszone elementaren Schwefel bei einem Molverhältnis von $C_1$—$C_{12}$-Alkylalkohol zu Schwefelwasserstoff zu elementarem Schwefel im Bereich von 2:1:0,5 bis 1:10:10 einspeist.

2. Verfahren nach Anspruch 1, dadurch gekennzeichnet, daß man das während der Reaktion erzeugte Alkylmercaptan wiedergewinnt und in die genannte Reaktionszone zurückführt.

3. Verfahren nach Anspruch 2, dadurch gekennzeichnet, daß der Alkylalkohol ein $C_1$—$C_4$-Alkylalkohol ist und daß die molare Geschwindigkeit 20 bis 200 ist.

4. Verfahren nach Anspruch 3, dadurch gekennzeichnet, daß der Alkohol, der Schwefelwasserstoff, der elementare Schwefel und das zurückgeführte Mercaptan in einem Molverhältnis von 1:1:0,5:2 bis 1:10:6:10 eingespeist werden, daß die Temperatur im Bereich von 250 bis 350°C liegt, daß der Druck im Bereich von 7,9 bis 32 bar (100 bis 450 psig) liegt und daß die molare Geschwindigkeit 50 bis 150 beträgt.

5. Verfahren nach Anspruch 3, dadurch gekennzeichnet, daß das als Nebenprodukt gebildete Dialkylsulfid in die genannte Reaktionszone zurückgeführt wird.

6. Verfahren nach Anspruch 3, dadurch gekennzeichnet, daß die als Nebenprodukt gebildeten Polysulfide in die genannte Reaktionszone zurückgeführt werden.

7. Verfahren nach Anspruch 3, dadurch gekennzeichnet, daß der Katalysator ein Katalysator vom X-, Y- oder L-Zeolith-Typ ist.

8. Verfahren nach Anspruch 3, dadurch gekennzeichnet, daß der Katalysator eine Heteropolysäure oder ihr Alkalimetall- oder Erdalkalimetallsalz ist, die bzw. das auf einem inerten Metalloxid-Träger aufgebracht ist.

9. Verfahren nach Anspruch 4, dadurch gekennzeichnet, daß der Katalysator ein Katalysator vom Y-Zeolith-Typ ist.

10. Verfahren nach Anspruch 4, dadurch gekennzeichnet, daß der Katalysator ein auf Aluminiumoxid aufgebrachtes Alkalimetallsalz einer Phosphorwolframsäure ist.

11. Verfahren zur kontinuierlichen Herstellung von Dimethyldisulfid durch kontinuierliche Einspeisung von Methanol, Schwefelwasserstoff, zurückgeführtem Mercaptan und einem inerten Gas in eine Reaktionszone bei einer Temperatur im Bereich von 250 bis 350°C, einem Druck von 7,9 bis 32 bar (100 bis 450 psig) und einer molaren Geschwindigkeit von 50 bis 150 und in Gegenwart eines Katalysators, ausgewählt aus der Gruppe bestehend aus Zeolithen vom Y-Typ, Aluminiumoxid, das Phosphorwolframsäure oder ihr Alkalimetallsalz trägt, und Aluminiumoxid, das Siliciumwolframsäure oder ihr Alkalimetallsalz trägt, dadurch gekennzeichnet, daß man kontinuierlich in die Reaktionszone elementaren Schwefel bei einem Molverhältnis von Methanol zu Schwefelwasserstoff zu elementarem Schwefel zu zurückgeführtem Mercaptan und zu einem inerten Gas von 1:1:0,5:2:1 bis 1:10:6:10:15 einspeist.

12. Verfahren nach Anspruch 11, dadurch gekennzeichnet, daß der Katalysator ein Katalysator vom protonierten Y-Zeolith-Typ ist.

13. Verfahren nach Anspruch 11, dadurch gekennzeichnet, daß der Katalysator ein Aluminiumoxid, das Alkalimetallsalz von Phosphorwolframsäure trägt, ist, wobei der Anteil des Salzes 1 bis 8%, bezogen auf das Katalysatorgewicht, beträgt.

**Revendications**

1. Procédé de préparation continue d'un disulfure de di(alkyle en $C_1$ à $C_{12}$), comprenant l'introduction continue dans une zone de réaction d'un alcool alkylique en $C_1$ à $C_{12}$ et de sulfure d'hydrogène dans une plage de température de 200 à 400°C à une pression allant de la pression atmosphérique à 42,3 bars (600 lb/in$^2$ au manomètre) et en présence d'un catalyseur solide en particules de manière à produire continuellement du disulfure de di(alkyle en $C_1$ à $C_{12}$), caractérisé par l'introduction continue dans la zone de réaction de soufre élémentaire dans des proportions molaires respectives d'alcool alkylique en $C_1$ à $C_{12}$, de sulfure d'hydrogène et de soufre élémentaire de 2/1/0,5 à 1/10/10.

2. Procédé suivant la revendication 1, dans lequel l'alkylmercaptan produit pendant la réaction est récupéré et recyclé dans la zone de réaction.

3. Procédé suivant la revendication 2, dans lequel l'alcool alkylique est un alcool alkylique en $C_1$ à $C_4$ et la vitesse molaire va de 20 à 200.

4. Procédé suivant la revendication 3, dans lequel l'alcool, le sulfure d'hydrogène, le soufre élémentaire et le mercaptan recyclé sont chargés dans des proportions molaires respectives de 1/1/0,5/2 à 1/10/6/10, la température va de 250 à 350°C, la pression va de 7,9 à 32 bars (100 à 450 lb/in$^2$ au manomètre) et la vitesse molaire va de 50 à 150.

5. Procédé suivant la revendication 3, dans lequel le sulfure de dialkyle obtenu comme sous-produit est recyclé dans la zone de réaction.

6. Procédé suivant la revendication 3, dans lequel les polysulfures formes comme sous-produits sont recyclés dans la zone de réaction.

7. Procédé suivant la revendication 3, dans lequel le catalyseur est une zéolite de type X, Y ou L.

8. Procédé suivant la revendication 3, dans lequel le catalyseur est un hétéropolyacide ou son sel de métal alcalin ou de métal alcalino-terreux, déposé sur un support formé d'un oxyde métallique inerte.

9. Procédé suivant la revendication 4, dans lequel le catalyseur est une zéolite de type Y.

10. Procédé suivant la revendication 4, dans lequel le catalyseur est un sel de métal alcalin d'acide phosphotungstique déposé sur de l'alumine.

11. Procédé de préparation continue de disulfure de diméthyle, comprenant l'introduction continue dans une zone de réaction de méthanol, de sulfure d'hydrogène, de mercaptan recyclé et d'un gaz inerte à une température allant de 250 à 350°C, à une pression de 7, 9 à 32 bars (100 à 450 lb/in$^2$ au manomètre) et à une vitesse molaire de 50 à 150, et en présence d'un catalyseur choisi dans le groupe comprenant des zéolites de type Y, de l'alumine portant de l'acide phosphotungstique ou son sel de métal alcalin et de l'alumine portant de l'acide silicotungstique ou son sel de métal alcalin, caractérisé par l'introduction continue dans la zone de réaction de soufre élémentaire dans des proportions molaires respectives de méthanol, de sulfure d'hydrogène, de soufre élémentaire, de mercaptan recyclé et de gaz inerte de 1/1/0,5/2/1 à 1/10/6/10/15.

12. Procédé suivant la revendication 11, dans lequel le catalyseur est une zéolite de type Y protonée.

13. Procédé suivant la revendication 11, dans lequel le catalyseur est une alumine portant un sel de métal alcalin d'acide phosphotungstique en proportion de 1 à 8% du sel sur la base du poids de catalyseur.

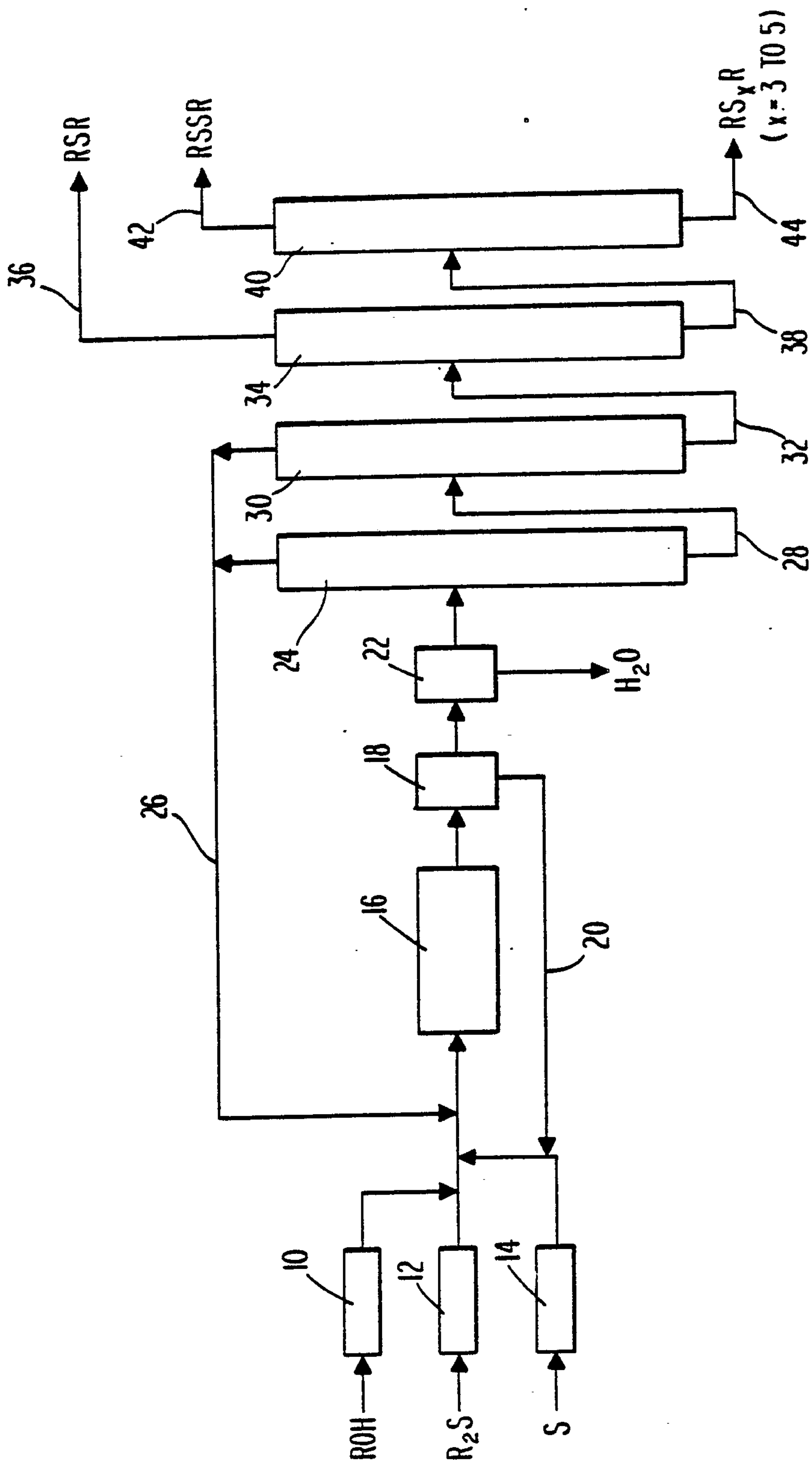
RSR
RSSR
$RS_xR$
(x=3 TO 5)
36
42
40
44
34
38
30
32
24
28
22
$H_2O$
18
26
16
20
10
12
14
ROH
$R_2S$
S